(19) Europäisches Patentamt
European Patent Office
Office européen des brevets

(11) Publication number: **0 085 370**
**B1**

(12) **EUROPEAN PATENT SPECIFICATION**

(45) Date of publication of patent specification: **16.09.87**

(21) Application number: **83100554.1**

(22) Date of filing: **22.01.83**

(51) Int. Cl.⁴: **A 61 K 7/043,** C 09 D 3/81, C 08 L 33/04 // (C08L33/04, 33:24)

(54) **Nitrocellulose free nail lacquer compositions.**

(30) Priority: **25.01.82 US 342397**

(43) Date of publication of application:
**10.08.83 Bulletin 83/32**

(45) Publication of the grant of the patent:
**16.09.87 Bulletin 87/38**

(84) Designated Contracting States:
**CH DE FR GB IT LI**

(56) References cited:
**GB-A-1 572 555**
**GB-A-2 073 229**
**US-A-3 277 056**

(73) Proprietor: **Wickhen Products, Inc.**
**Big Pond Road**
**Huguenot New York 12746 (US)**

(72) Inventor: **Abrutyn, Eric Steven**
**9 Westminster Drive**
**Middletown New York 10940 (US)**

(74) Representative: **Bauer, Wulf, Dr.**
**Wolfgang-Müller-Strasse 12**
**D-5000 Köln 51 (Marienburg) (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 085 370 B1

**Description**

The invention relates to nitrocellulose free nail lacquer compositions. While nitrocellulose is the film former of choice, it has numerous disadvantages. For example, nitrocellulose discolors with age, is prone to undergo sharp viscosity changes rendering nail lacquer compositions difficult to apply, and it can be difficult to dry to a hard film. Furthermore, care must be taken to insure that nitrocellulose used in formulating nail lacquers is neutral, i.e., acid free, because the presence of free acid could cause damage to fingernails and the cuticle, as well as have a deleterious effect on colorants present in nail lacquers.

Of course, those skilled in the art recognize that nitrocellulose must be produced and handled with great caution and care. The danger of explosion and fire inherent in nitrocellulose production and formulation of compositions containing nitrocellulose is responsible in large measure for the fact that there are very few manufacturers of this product, and only a few formulators of nail lacquers.

The art has frequently sought substitutes for nitrocellulose as a film former for nail lacquers. Attempts to find substitutes for nitrocellulose have not been successful, because, despite its many drawbacks, nitrocellulose provides nail lacquer compositions with an unusual combination of desirable properties such as toughness, durability and solvent release, and it produces waterproof and atmospherically stable films. For examples of such attempts see U.S. Patents No. 3,840,490; 3,864,294; 3,927,203; 3,298,113 and 4,240,450. A nail lacquer composition in which nitrocellulose is at least partially replaced is known from GB—A—2 073 229, which discloses anhydrous nail varnishes containing a copolymer (terpolymer of higher polymer) resulting from copolymerization of at least one unsaturated polar monomer of a given formula, at least one methacrylate monomer of a given formula, and at least one alkyl ($C_1$—$C_{18}$) acrylate monomer.

It was noted, however, that a non-nitrocellulose lacquer cannot always be obtained through the use of the teaching of GB—A—2 073 229. The object of this invention is to provide nail lacquer compositions which do not contain nitrocellulose, but which have characteristics and properties which are equivalent to conventional nail lacquer compositions containing nitrocellulose.

A further object of the invention is to provide nitrocellulose free nail lacquer compositions which, when applied to nails, impart a durable film with high gloss characteristics, flexibility, breathability and excellent adhesion.

The present invention relates to a nitrocellulose free nail lacquer composition comprising copolymers of hydrophobic and hydrophilic monomers, wherein said hydrophobic monomers are a mixture of at least two different esters of α,β unsaturated carboxylic acids, said esters having the formula:

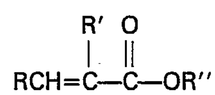

wherein:

R is selected from H, $CH_3$ and $C_2H_5$;

R′ is selected from H and $C_1$ to $C_4$ alkyl radicals; and

R″ is a member selected from:

(i) $CH_3$ to $C_4H_9$ straight chain alkyl radicals,

(ii) saturated or unsaturated cyclic alkyl radicals containing up to 20 carbon atoms,

(iii) $C_3R_7$ to $C_{16}H_{37}$ branched alkyl or $C_5H_{11}$ to $C_{22}H_{45}$ straight chain alkyl radicals, and

(iv) alkoxy or aryloxy alkyl radicals; and,

R″ is different for each of said esters; and,

said hydrophilic monomers are comprised of N-oxo substituted acrylamides of the formula:

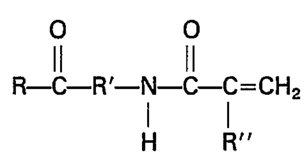

wherein:

R is hydrogen or $C_1$—$C_{10}$ alkyl group, R′ is ethylene or a $C_1$—$C_{10}$ alkyl-substituted ethylene radical, R″ is selected from hydrogen or $CH_3$ and said copolymers of hydrophobic and hydrophilic monomers being in a suitable carrier;

said copolymers comprising from 70% to 90% of said hydrophobic monomers and from 5 to 30% of said hydrophilic monomers, said percentages being by weight and based on the weight of the copolymer, the copolymers being present in said nail lacquer composition in an amount from 10 to 40%, by weight, based on the weight of said composition.

Especially preferred nail lacquer compositions of this invention include those wherein the hydrophilic monomers comprise mixtures of N-oxo substituted acrylamides and methacrylamides.

Especially preferred nail lacquer compositions of this invention include those wherein the hydrophobic monomers comprise mixtures of esters, comprising from:

(i) about 30%, by weight, straight chain alcohols;

(ii) about 40%, by weight, saturated or unsaturated cyclic alcohols;
(iii) about 15% by weight, branched alkyl or straight chain higher alkyl alcohols; and
(iv) about 10%, by weight, alkoxy or aryloxy alcohols.

The esters formed with the straight chain alcohols defined above which are useful as hydrophobic monomers in forming the nail lacquer compositions of this invention include methyl acrylate, methyl methacrylate, ethyl acrylate, ethyl methacrylate, propyl acrylate, propyl methacrylate, butyl acrylate or butyl methacrylate. Hydrophobic monomers of this type are thought to impart hardness to films provided by the nail lacquer compositions of this invention. Esters formed with saturated or unsaturated cyclic alcohols described above which are useful as hydrophobic monomers for preparing the nail lacquer compositions of this invention include cyclohexyl acrylate, cyclohexyl methacrylate, benzyl acrylate, benzyl methacrylate, isobornyl acrylate, isobornyl methacrylate, adamantyl acrylate, adamantyl methacrylate, furfuryl acrylate and furfuryl methacrylate. Hydrophobic monomers of this type are thought to impart gloss and hardness to films provided by the nail lacquer compositions of this invention. Esters of branched alkyl or straight chain higher alkyl alcohols described above which are useful as hydrophobic monomers for preparing the nail lacquer compositions of this invention include 2-ethylhexyl acrylate, 2-ethylhexyl methacrylate, isobutyl acrylate, isobutyl methacrylate, isooctyl acrylate, isooctyl methacrylate, dodecylacrylate, dodecyl methacrylate, octadodecyl acrylate and octadodecyl methacrylate. Hydrophobic monomers of this type are thought to impart internal plasticization, pliability and breathability to nail lacquer compositions of this invention. Esters of alkoxy or aryloxy alkyl alcohols useful as hydrophobic monomers for preparing the nail lacquer compositions of this invention include methoxyethyl acrylate, methoxyethyl methacrylate, ethoxyethyl acrylate, ethoxyethyl methacrylate, propoxyethyl acrylate, propoxyethyl methacrylate, butoxyethyl acrylate, butoxyethyl methacrylate, phenoxyethyl acrylate, and phenoxyethyl methacrylate. Hydrophobic monomers of this type are thought to impart gloss, flexibility and adhesion to films provided by the nail lacquer compositions of this invention.

While it is preferred that the hydrophobic monomers useful in forming the copolymers be comprised of a mixture of each of the four classes of esters described above, it will be appreciated that the hydrophobic monomer portion of the copolymers may be comprised of a mixture of any two or more of the four classes of esters. When the monomers comprising the hydrophobic portion consist of monomers from less than each of the four classes of esters described above, it is generally preferred that monomers from either of classes (i) or (ii) be present in the hydrophobic monomer portion.

Hydrophilic monomers are copolymerized with the hydrophobic monomers described above in forming the nail lacquer compositions of this invention. "Hydrophilic" monomers in the context of this invention are meant to encompass those monomers which impart dual functionality to the nail lacquer compositions whereby such compositions will interact with receptor sites of human nails providing for nail lacquer films which demonstrate excellent adhesion, durability, breathability and high gloss. The hydrophilic monomers used in the invention are (i) N-oxosubstituted acrylamides or methacrylamides. The monomers (ii) α-β unsaturated carboxylic acids, and (iii) hydroxyl alkyl acrylates and methacrylates may be also used, but only optionally and additionally to monomers (i). Monomers (i) useful in preparing the nail lacquer compositions of this invention include diacetone acrylamide and its homologs as described in U.S. Patent No. 3,277,056, dimethyl acrylamide, butyl acrylamide, octyl acrylamide, and isobutyl methyl acrylamide. The acrylamide hydrophilic monomers are thought to impart adhesion and breathability to films formed with the nail lacquer compositions of this invention.

The α-β unsaturated carboxylic acid hydrophilic monomers useful in forming the nail lacquer compositions of this invention include acrylic acid, methacrylic acid, crotonic acid, itaconic acid, maleic acid and fumaric acid. The hydrophilic monomers of this type are thought to impart adhesion to films formed from the nail lacquer compositions of this invention.

The hydroxyalkyl acrylates and methacrylates useful in forming the nail lacquer compositions of this invention include hydroxyethyl acrylate and methacrylate, diethylene glycol monoacrylate and monomethacrylate, triethylene glycol monoacrylate and monomethacrylate, tetraethylene glycol monoacrylate and monomethacrylate, polyethylene glycol monoacrylate and monomethacrylate of molecular weight up to 1000, linear or branched hydroxypropyl acrylate and methacrylate, dipropylene glycol monoacrylate and monomethacrylate, tripropylene glycol monoacrylate and monomethacrylate, tetrapropylene glycol monoacrylate and monomethacrylate, and polypropylene glycol monoacrylate and monomethacrylate of molecular weight up to 1000. Hydrophilic monomers of this type are thought to provide improved permeability to films provided by the nail lacquer compositions of the invention.

The hydrophilic monomers useful in forming the nail lacquer compositions of the invention comprise mixtures of from 50 to 75%, preferably 60 to 65%, by weight, of the acrylamides described above and from 25 to 50%, preferably 35 to 40%, by weight, of the α-β unsaturated carboxylic acids described above. Hydroxyalkyl acrylates and methacrylates may be included in the nail lacquer compositions of the invention in a ratio of 1:1 with the other hydrophilic monomers described herein.

A particular significant aspect of the invention lies in the fact that the new nail lacquer compositions represent a balance of hydrophobic and hydrophilic characteristics which impart dual functionality to the films obtained on application of the compositions to the nails. In the course of this invention, it has been found that the dual functional nature of the copolymer systems comprising the nail lacquer compositions provides nail lacquer films which have high gloss, flexibility, excellent adhesive properties, breathability,

and excellent water resistant properties. It is a particularly significant aspect of this invention that such properties are provided by nail lacquer compositions which do not contain nitrocellulose, on the one hand, but which, on the other, do contain hydrophilic monomers, a component which those skilled in the art would ordinarily expect to be incompatible with nail lacquer films demonstrating excellent water resistant properties.

Particularly preferred nail lacquer compositions of the invention are those in which copolymers of hydrophobic and hydrophilic monomers are present in an amount of from 30 to 40%, by weight, based on the weight of the composition.

A particularly desirable attribute of the present invention lies in the fact that it is possible to incorporate colorants into the nail lacquer compositions of the invention without significant risk of explosion or fire which is found when colorants are combined with conventional nail lacquers containing nitrocellulose.

Liquid carriers for the nail lacquer compositions of the invention are preferably selected from materials which are solvents for the copolymers. Suitable solvents include ethyl acetate, amyl acetate, butyl acetate, butyl cellosolve acetate, cellosolve acetate, methyl cellosolve acetate, acetone, methyl ethyl ketone, methyl isobutyl ketone, butyl cellosolve, cellosolve, methyl cellosolve, ethyl alcohol, isopropyl alcohol, butyl alcohol, toluene, and xylene. Other solvents may also be used as carriers as will be appreciated by those in the art. Suitable carriers for the nail lacquer compositions comprise any one or a mixture of any of the foregoing solvents in any proportion. A particularly preferred carrier for the nail lacquer compositions of the invention is a 1:1 mixture of butyl acetate and isopropyl alcohol.

When applied to nails, primarily human nails, the nail lacquer compositions of the invention provide films which are the equivalent of conventional nitrocellulose containing nail lacquers. The nail lacquer compositions of the invention are compatible with conventional plasticizers, colorants, suspending agents, viscosity builders, additional film forming resins and fillers. Plasticizers include those well known in the art such as isopropyl alcohol fatty acid esters, $C_8$ alcohol fatty acid esters, organic succinates, organic phthalates, organic adipates, camphor, and castor oil. Colorants include those well known in the art, particularly pigments, and are included in nail lacquer compositions used to impart color to the nails. Such components can be combined with nail lacquer compositions of the invention by means well known to those skilled in the art to provide colored nail lacquer compositions of any desired hue. Suspending agents are used in nail lacquer compositions containing colorants, and serve to maintain the colorant in suspension in the nail lacquer composition. Useful viscosity builders include tetraethylene glycol dimethacrylate, trimethylol propane trimethacrylate, tetrahydrofurfuryl methacrylate, and allyl methacrylate. Such components are typically incorporated in nail lacquer compositions of the invention in an amount of from 0.1 to 0.5%, preferably about 0.25%, by weight, based on the weight of the nail lacquer composition.

Useful additional film forming resins which may be incorporated in the nail lacquer compositions of this invention include aryl sulfanamide/formaldehyde, sucrose acetate isobutyrate, sucrose benzoate and diethylene/dipropylene glycol dibenzoate which may be incorporated in an amount of from 0% to 20%, by weight, preferably 5 to 10% by weight, based on the weight of the compositions. Whether clear or colored, the nail lacquer compositions of the invention have a high solids content, and thus, provide an eminently acceptable film on nails with a one coat application.

In addition to empirical comparisons of films provided by the nail lacquer compositions of the invention with those provided by conventional nitrocellulose containing nail lacquers, it is possible to compare properties of films provided by the two types of compositions by applying films of the respective nail lacquer compositions to black glass plates or slides, allowing the films to dry at ambient conditions followed by visual, mechanical or instrumental evaluations of the film properties.

The invention also provides a process for production of nitrocellulose free nail lacquer compositions which comprises combining hydrophobic and hydrophilic monomers in a suitable carrier, polymerizing said monomers producing copolymer nail lacquer compositions in which said copolymer is present in an amount sufficient to form a high gloss, flexible, breathable, well adhering, waterproof film when applied to nails.

Generally, the process involves mixing the hydrophobic and hydrophilic monomers in a suitable solvent-carrier selected from those set forth hereinabove, to form a uniform mixture, and inducing polymerization. Polymerization may be induced by conventional initiators such as peroxides and the like, or by irradiation or redox systems. Polymerization usually occurs at temperatures between 0° to 120°C, and preferably at the reflux temperature of the solvent carrier.

The time and temperature of polymerization may be varied in accord with techniques well known in the art. Polymerization results in a clear solution in which the copolymer is present in an amount of from 10% to 40%, by weight, based on the weight of the composition.

The nail lacquer compositions of this invention offer numerous advantages over conventional, nitrocellulose containing nail lacquer compositions. For example, nitrocellulose is difficult to dissolve. Frequently, aromatic solvents including benzene and toluene are required to dissolve nitrocellulose in conventional nail lacquer formulations. The nail lacquer compositions of this invention do not require use of such potentially toxic solvents and instead, require use of simple solvents such as alcohols. Moreover, pigments and colorants may be incorporated in the nitrocellulose free nail lacquer compositions of the

invention without the great fear of fire and explosion inherent when pigments are milled in nitrocellulose base nail lacquer compositions.

The invention is further described by reference to the following examples which are intended to be illustrative of the invention.

Example 1

A nail lacquer composition was prepared by mixing the following ingredients in a 3 l, three neck round bottom flask equipped with a stirrer, thermometer and condenser, and heating the mixture at the reflux temperature of the solvent from 2 to 24 hours.

| Ingredient | Amount (grams) |
|---|---|
| Diacetone acrylamide | 46.5 |
| Isobornyl methacrylate | 178.5 |
| Ethyl methacrylate | 150 |
| Phenoxyethyl methacrylate | 55 |
| Methacrylic acid | 30 |
| 2-Ethylhexyl methacrylate | 65 |
| Butyl acetate | 395 |
| Isopropanol | 395 |
| Benzoyl peroxide | 2.85 |

The resulting copolymer was a clear, viscous liquid which when applied to nails provided a high gloss, long lasting film which was the equivalent of conventional nitrocellulose containing nail lacquers.

Example 2

Using the equipment and following the procedure of Example 1, the following ingredients were combined and polymerized.

| Ingredient | Amount (grams) |
|---|---|
| Diacetone acrylamide | 93 |
| Isobornyl methacrylate | 371 |
| Methyl methacrylate | 302 |
| Phenoxyethyl acrylate | 69 |
| 2-Ethylhexyl methacrylate | 174 |
| Methacrylic acid | 47 |
| Tetrahydrofurfuryl methacrylate | 2.5 |
| Butyl acetate | 1455 |
| Benzoyl peroxide | 5.75 |

The resulting copolymer was a clear, viscous liquid which provided a film when applied to nails which was the equivalent of conventional nitrocellulose containing nail lacquers.

Example 3

A nail lacquer composition was prepared by mixing the following ingredients in a 0.5 l pressure bottle and heating the mixture at about 90°C for about 24 hours with constant agitation.

| Ingredient | Amount (grams) |
|---|---|
| Diacetone acrylamide | 4.25 |
| Dimethyl acrylamide | 4.25 |
| Ethyl methacrylate | 25.5 |
| Cyclohexyl methacrylate | 34 |
| Phenoxyethyl methacrylate | 8.5 |
| Methacrylic acid | 4.25 |
| Tetrahydrofurfuryl methacrylate | 0.2 |
| Butyl acetate | 78.5 |
| Isopropanol | 78.5 |
| Benzoyl peroxide | 2.2 |

The resulting copolymer was a clear, viscous liquid which provided a film when applied to nails which was the equivalent of conventional nitrocellulose containing nail lacquers.

Example 4

Using equipment and following the procedure of Example 3, the following ingredients were combined and polymerized.

| Ingredient | Amount (grams) |
|---|---|
| Diacetone acrylamide | 4.25 |
| Dimethyl acrylamide | 4.25 |
| Butyl methacrylate | 25.5 |
| Cyclohexyl methacrylate | 34 |
| Phenoxyethyl methacrylate | 8.5 |
| Methacrylic acid | 4.25 |
| Tetrahydrofurfuryl methacrylate | 0.2 |
| Butyl acetate | 78.5 |
| Isopropanol | 78.5 |
| Benzoyl peroxide | 2.2 |

The resulting copolymer was a clear, viscous liquid which provided a film when applied to nails which was the equivalent of conventional nitrocellulose containing nail lacquers.

Example 5

Using the equipment and following the procedure of Example 3, the following ingredients were combined and polymerized at a temperature of about 80°C.

| Ingredient | Amount (grams) |
|---|---|
| Diacetone acrylamide | 8.75 |
| Hydroxyethyl methacrylate | 8.75 |
| Cyclohexyl methacrylate | 36.75 |
| Ethyl methacrylate | 17.50 |
| Isodecyl methacrylate | 8.75 |
| Methacrylic acid | 7 |
| Butyl acetate | 81.25 |
| Ethyl acetate | 81.25 |
| Benzoyl peroxide | 1.75 |

The resulting copolymer was a clear, viscous liquid which provided a film when applied to nails which was the equivalent of conventional nitrocellulose containing nail lacquers.

Example 6

Using the equipment and following the procedure of Example 3, the following ingredients were combined and polymerized at a temperature of about 120°C.

| Ingredient | Amount (grams) |
|---|---|
| N-(iso-butoxymethyl)acrylamide | 8.5 |
| Isobornyl methacrylate | 34.4 |
| Methyl methacrylate | 34.4 |
| Methacrylic acid | 4.25 |
| Tetrahydrofurfuryl methacrylate | 0.17 |
| Butyl acetate | 158 |
| t-Butyl peroctoate | 1.9 ml |

The resulting copolymer was a clear, viscous liquid which provided a film when applied to nails which was the equivalent of conventional nitrocellulose containing nail lacquers.

Example 7

Using the equipment and following the procedure of Example 3, the followinig ingredients were combined and polymerized at a temperature of about 120°C.

| Ingredient | Amount (grams) |
|---|---|
| Diacetone acrylamide | 4.25 |
| Octyl acrylamide | 5.25 |
| Methyl methacrylate | 4.42 |
| Isobornyl methacrylate | 34.42 |
| Methacrylic acid | 4.25 |
| Tetrahydrofurfuryl methacrylate | 0.17 |
| Butyl acetate | 158 |
| t-Butyl peroctoate | 1.9 ml |

The resulting copolymer was a clear, viscous liquid which provided a film when applied to nails which was the equivalent of conventional nitrocellulose containing nail lacquers.

Example 8

Using the equipment and following the procedure of Example 3, the following ingredients were combined and polymerized at a temperature of about 120°C.

| Ingredient | Amount (grams) |
|---|---|
| Diacetone acrylamide | 8.5 |
| Methyl methacrylate | 34.4 |
| Methoxyethyl methacrylate | 34.4 |
| Methacrylic acid | 4.25 |
| Tetrahydrofurfuryl methacrylate | 0.17 |
| Butyl acetate | 158 |
| t-Butyl peroctoate | 1.9 |

The resulting copolymer was a clear, viscous liquid which provided a film when applied to nails which was the equivalent of conventional nitrocellulose containing lacquers.

Example 9

A nail lacquer composition was prepared by combining and mixing 65.5 parts of the base polymer solution of Example 1, 11.5 parts sucrose benzoate, 5 parts castor oil and 18 parts isopropanol until a clear, uniform composition was obtained. The presence of a plasticizer such as castor oil provides improved pliability of the film.

Example 10

A nail lacquer composition was prepared by combining and mixing 75 parts base polymer solution of Example 1, 10 parts aryl sulfonamide/formaldehyde (Santolite® MHP/Monsanto), and 15 parts isopropanol until a clear, uniform composition was obtained. The composition was the equivalent of nitrocellulose nail lacquer compositions when applied to nails.

Example 11

A nail lacquer composition was prepared by combining and mixing 67.5 parts of base polymer solution of Example 1, 11.8 parts of sucrose acetate isobutyrate, 18.5 parts of isopropanol, 1 part castor oil and 1 part Timiron® MP-1005 (Rona Pearl, Bayonne, N.J.) pigment. Mixing continued until the pigment was uniformly dispersed and the resultant pigmented nail lacquer when applied to nails was the equivalent of conventional nitrocellulose containing nail lacquers.

Example 12

A nail lacquer composition was prepared by combining and mixing 50 parts of base polymer solution of Example 1, 5 parts of dipropylene glycol dibenzoate, 1 part isopropyl myristate and 44 parts isopropanol. The resulting copolymer was a clear, viscous liquid which provided a film when applied to nails which was the equivalent of conventional nitrocellulose containing lacquers.

**Claims**

1. A nitrocellulose free nail lacquer composition comprising copolymers of hydrophobic and hydrophilic monomers, wherein said hydrophobic monomers are a mixture of at least two different esters of α,β unsaturated carboxylic acids, said esters having the formula:

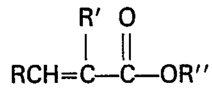

wherein:

R is selected from H, $CH_3$ and $C_2H_5$;

R′ is selected from H and $C_1$ to $C_4$ alkyl radicals; and

R″ is a member selected from:

(i) $CH_3$ to $C_4H_9$ straight chain alkyl radicals,

(ii) saturated or unsaturated cyclic alkyl radicals containing up to 20 carbon atoms,

(iii) $C_3H_7$ to $C_{16}H_{37}$ branched alkyl or $C_5H_{11}$ to $C_{22}H_{45}$ straight chain alkyl radicals, and

(iv) alkoxy or aryloxy alkyl radicals; and,

R″ is different for each of said esters; and,

said hydrophilic monomers are comprised of N-oxo substituted acrylamides of the formula:

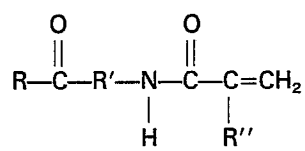

wherein:

R is hydrogen or $C_1$—$C_{10}$ alkyl group,

R′ is ethylene or a $C_1$—$C_{10}$ alkyl-substituted ethylene radical,

R″ is selected from hydrogen or $CH_3$ and

said copolymers of hydrophobic and hydrophilic monomers being in a suitable carrier;

said copolymers comprising from 70% to 90% of said hydrophobic monomers and from 5 to 30% of said hydrophilic monomers, said percentages being by weight and based on the weight of the copolymer, the copolymers being present in said nail lacquer composition in an amount from 10 to 40%, by weight, based on the weight of said composition.

2. The nail lacquer composition of Claim 1 wherein said hydrophobic monomers comprise a mixture of any two or more of the esters of classes (i), (ii), (iii) and (iv), and preferably a mixture of the esters of classes (i) and (ii).

3. The nail lacquer composition of Claim 1 or 2 wherein said hydrophilic monomers comprise mixtures of N-oxo substituted acrylamides and methacrylamides.

4. The nail lacquer composition of Claim 3 wherein said hydrophilic monomer mixture also contains hydroxyalkyl acrylates and methacrylates.

5. The nail lacquer composition of Claim 3 wherein said hydrophilic monomers comprise mixture of from 50 to 75%, preferably 75%, by weight, of said N-oxo substituted acrylamides and from 25 to 50%, preferably 25%, by weight, of said α,β unsaturated carboxylic acids.

6. The nail lacquer composition of one of the preceding claims wherein said carrier is a solvent for said copolymers.

7. The nail lacquer composition of Claim 6 wherein said solvent is selected from ethyl acetate, amyl acetate, butyl acetate, butyl cellosolve acetate, cellosolve acetate, methyl cellosolve acetate, acetone, methyl ethyl ketone, methyl isobutyl ketone, butyl cellosolve, cellosolve, methyl cellosolve, ethyl alcohol, isopropyl alcohol, butyl alcohol, toluene, and xylene.

8. The nail lacquer composition of Claim 6 or 7 wherein said solvent is a 1:1 mixture of butyl acetate and isopropyl alcohol.

9. The nail lacquer composition of one of the preceding claims colorants, suspending agents and fillers, and/or further comprising at least one of an additional film forming resin and a plasticizer.

10. The nail lacquer composition of Claim 9 including viscosity builders selected from ethylene glycol dimethacrylate, tetraethylene glycol dimethacrylate, trimethylol propane trimethacrylate, tetrahydrofurfuryl methacrylate and allyl methacrylate in an amount of from 0.1 to 0.5%, by weight, based on the weight of said composition.

11. The nail lacquer composition of claim 3 wherein said copolymers of hydrophobic and hydrophilic monomers are present in the amount from 30 to 40%, by weight, based on the weight of the composition.

12. The nail lacquer composition of one of the preceding claims wherein said hydrophobic monomers comprise a major portion of said copolymers.

13. A nitrocellulose free nail lacquer composition comprising copolymers of hydrophobic and hydrophilic monomers said hydrophobic monomers being a mixture of at least two different esters of α,β unsaturated carboxylic acids, said esters being selected from:

(i) methyl acrylate, methyl methacrylate, ethyl acrylate, ethyl methacrylate, propyl acrylate, propyl methacrylate, butyl acrylate, butyl methacrylate,

(ii) cyclohexyl acrylate, cyclohexyl methacrylate, benzyl acrylate, benzyl methacrylate, isobornyl acrylate, isobornyl methacrylate, adamantyl acrylate, adamantyl methacrylate, furfuryl acrylate, furfuryl methacrylate.

(iii) 2-ethylhexyl acrylate, 2-ethylhexyl methacrylate, isobutyl acrylate, isobutyl methacrylate, isooctyl acrylate, isooctyl methacrylate, dodecylacrylate, dodecyl methacrylate, octadodecyl acrylate, octadodecyl methacrylate, isodecyl acrylate, isodecyl methacrylate,

(iv) methoxyethyl acrylate, methoxyethyl methacrylate, ethoxyethyl acrylate, ethoxyethyl methacrylate, propoxyethyl acrylate, propoxyethyl methacrylate, butoxyethyl acrylate, butoxyethyl methacrylate, phenoxyethyl acrylate, and phenoxyethyl methacrylate; and,

said hydrophilic monomers being comprised of N-oxo substituted acrylamides, said acrylamides are selected from diacetone acrylamide, dimethyl acrylamide, butyl acrylamide, octyl acrylamide, isobutyl methyl acrylamide, and N-(iso-butoxymethyl) acrylamide; and

said copolymers of hydrophobic and hydrophilic monomers being in a suitable carrier;

said copolymers comprising from 70% to 90% of said hydrophobic monomers and from 5 to 30% of said hydrophilic monomers, said percentages being by weight, and based on the weight of the copolymer, the copolymers being present in said nail lacquer composition in an amount from 10 to 40%, by weight, based on the weight of said composition.

**Patentansprüche**

1. Ein nitrocellulosefreies Nagellackmittel mit Copolymeren hydrophober und hydrophiler Monomere, wobei die hydrophoben Monomere eine Mischung von zumindest zwei unterschiedlichen Estern von α,β ungesättigten Carbonsäuren sind, diese Ester haben die Formel

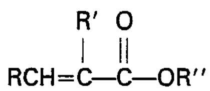

wobei:

R ausgewählt ist aus H, $CH_3$ und $C_2H_5$;

R' ausgewählt ist aus H und $C_1$ bis $C_4$ Alkylradikalen; und

R'' ein Glied ist, das ausgewählt ist aus

(i) $CH_3$ bis $C_4H_9$ geradkettigen Alkylradikalen,

(ii) gesättigten oder ungesättigten cyclischen Alkylradikalen mit bis zu 20 Carbonatomen,

(iii) $C_3H_7$ bis $C_{16}H_{37}$ verzweigten Alkyl oder $C_5H_{11}$ bis $C_{22}H_{45}$ geradkettigen Alkylradikalen und

(iv) Alkoxyl- oder Aryloxyl-Alkylradikalen; und

R'' unterschiedlich für jeden dieser Ester ist; und die hydrophilen Monomere N-Oxo substituierte Acrylamide der Formel

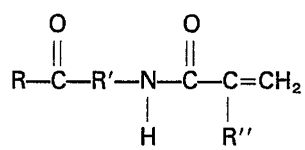

umfassen, wobei R Wasserstoff oder eine $C_1$—$C_{10}$ Alkylgruppe ist,

R' Äthylen oder ein $C_1$ bis $C_{10}$ alkylsubstituiertes Äthylenradikal ist,

R'' ausgewählt ist aus Wasserstoff oder $CH_3$ und die Copolymere von hydrophoben und hydrophilen Monomeren sich in einem geeigneten Trägermedium befinden;

diese Copolymere enthalten 70 Prozent bis 90 Prozent dieser hydrophoben Monomere und 5 bis 30 Prozent der hydrophilen Monomere, die Prozentangaben sind in Gewichtsprozent und basieren auf dem Gewicht des Copolymers, die Copolymere sind im Nagellackmittel in einer Menge von 10 bis 40 Gewichts-Prozenten vorhanden, bezogen auf das Gewicht des Mittels.

2. Nagellackmittel nach Anspruch 1, wobei die hydrophoben Monomere eine Mischung von beliebigen zwei oder mehr Estern der Klassen (i), (ii), (iii) und (iv), und vorzugsweise eine Mischung von Estern der Klassen (i) und (ii), aufweisen.

3. Nagellackmittel nach Anspruch 1 oder 2, wobei die hydrophilen Monomere Mischungen von N-oxo substituierten Acrylamiden und Methacrylamiden aufweisen.

4. Nagellackmittel nach Anspruch 3, wobei die hydrophile Monomermischung auch Hydroxyalkylacrylate und -methacrylate enthält.

5. Nagellackmittel nach Anspruch 3, wobei die hydrophilen Monomere eine Mischung von 50 bis 75 Gewichts-Prozent, vorzugsweise 75 Gewichts-Prozent, an den N-oxo substituierten Acrylamiden und von 25 bis 50 Gewichts-Prozent, vorzugsweise 25 Gewichts-Prozent an den α,β ungesättigten Carbonsäuren aufweisen.

6. Nagellackmittel nach einem der vorhergehenden Ansprüche, wobei das Trägermittel ein Lösungsmittel für die Copolymere ist.

7. Nagellackmittel nach Anspruch 6, wobei das Lösungsmittel ausgewählt ist aus Äthylacetat, Amylacetat, Butylacetat, Butylcellosolveacetat, Cellosolveacetat, Methylcellosolveacetat, Aceton,

Methyläthylketon, Methylisobutylketon, Butylcellosolve, Cellosolve (Äthylglykol), Methylcellosolve, Äthylalkohol, Isopropylalkohol, Butylalkohol, Toluen und Xylen.

8. Nagellackmittel nach Anspruch 6 oder 7, wobei das Lösungsmittel eine 1:1 Mischung von Butylacetat und Isopropylalkohol ist.

9. Nagellackmittel nach einem der vorangegangenen Ansprüche mit Farbstoffen, Suspensionsmitteln und Füllern und/oder weiterhin mit mindestens einem zusätzlichen filmbildenden Harz oder einem Plastifikator.

10. Nagellackmittel nach Anspruch 9 mit Viskositätsbildnern ausgewählt aus Äthylenglykoldimethacrylat, Tetraäthylenglykoldimethylacrylat, Trimethylolpropantrimethacrylat, Tetrahydrofurfurylmethacrylat und Allylmethacrylat in einer Menge von 0,1 bis 0,5 Gewichts-Prozenten, basierend auf dem Gewicht des Mittels.

11. Nagellackmittel nach Anspruch 3, wobei die Copolymere der Hydrophoben und hydrophilen Monomere in einer Menge von 30 bis 40 Gewichts-Prozenten vorliegen, bezogen auf das Gewicht des Mittels.

12. Nagellackmittel nach einem der vorhergehenden Ansprüche, wobei die hydrophoben Monomere einen Hauptanteil der Copolymere ausmachen.

13. Ein nitrocellulosefreies Nagellackmittel mit Copolymeren hydrophober und hydrophiler Monomere, wobei die hydrophoben Monomere eine Mischung von zumindest zwei unterschiedlichen Estern α,β ungesättigter Carbonsäuren sind, wobei die Ester ausgewählt sind aus:

(i) Methlacrylat, Methylmethacrylat, Äthylacrylat, Äthylmethacrylat, Propylacrylat, Propylmethacrylat, Butylacrylat, Butylmethacrylat,

(ii) Cyclohexylacrylat, Cyclohexylmethacrylat, Benzylacrylat, Benzylmethacrylat, Isobornylacrylat, Isobornylmethacrylat, Adamantylacrylat, Adamantylmethacrylat, Furfurylmethacrylat, Furfurylmethacrylat,

(iii) 2-Äthylhexylacrylat, 2-Äthylhexylmethacrylat, Isobutylacrylat, Isobutylmethacrylat, Isooctylacrylat, Isooctylmethacrylat, Dodecylacrylat, Dodecylmethacrylat, Octadodecylacrylat, Octadodecylmethacrylat, Isodecylacrylat Isodecylmethacrylat,

(iv) Methoxyäthylacrylat, Methoxyäthylmethacrylat, Äthoxyäthylacrylat, Äthoxyäthylmethacrylat, Propoxyäthylacrylat, Propoxyäthylmethacrylat, Butoxyäthylacrylat, Butoxyäthylmethacrylat, Phenoxyäthylacrylat, und Phenoxyäthylmethacrylat, und

die hydrophilen Monomere N-oxo substituierte Acrylamide aufweisen, diese Acrylamide sind ausgewählt aus Diacetonacrylamid, Dimethylacrylamid, Butylacrylamid, Octylacrylamid, Isobutylmethylacrylamid, und N-(Iso-Butoxymethyl)-Acrylamid, und diese Copolymere hydrophobe und hydrophile Monomere in einem geeigneten Trägermedium sind, wobei die Copolymere 70 bis 90 Prozent der hydrophoben Monomere und 5 bis 30 Prozent der hydrophilen Monomere aufweisen, die Prozentangaben sind in Gewichts-Prozenten und basieren auf dem Gewicht des Copolymers, das Copolymer ist in dem Nagellackmittel in einer Menge von 10 bis 40 Gewichts-Prozenten, bezogen auf das Gewicht des Mittels, vorhanden.

## Revendications

1. Une composition der vernis à ongles sans nitrocellulose qui comprend des copolymères de monomères hydrophobes et de monomères hydrophiles, dont les monomères hydrophobes sont constitués par un mélange d'au moins deux esters différents d'acides carboxyliques α,β-insaturés, de formule:

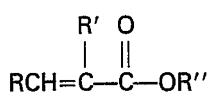

dans laquelle

R représente l'hydrogène ou le groupe méthyle ou éthyle;

R' l'hydrogène ou un alkyle en $C_1$ à $C_4$; et

R'' est choisi parmi:

(1) des alkyles à chaîne droite de $CH_3$ à $C_4H_9$;

(2) des cycloalkyles saturés ou insaturés pouvant avoir jusqu'à 20 atomes de carbone;

(3) des alkyles ramifiés de $C_3H_7$ à $C_{16}H_{37}$ ou des alkyles à chaîne droite de $C_5H_{11}$ à $C_{22}H_{45}$; et

(4) des radicaux alcoxy ou aryloxy alkyles;

R'' étant différent pour chacun de ces esters; et

les monomères hydrophiles sont formés de N-oxo-acrylamides de formule:

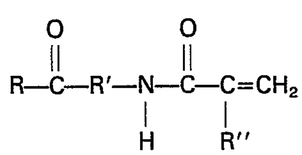

dans laquelle

R représente l'hydrogène ou un alkyle en $C_1$ à $C_{10}$;

R' le radical éthylène ou un radical éthylène portant un alkyle en $C_1$ à $C_{10}$; et

R'' l'hydrogène ou le groupe $CH_3$,

ces copolymères de monomères hydrophobes et de monomères hydrophiles étant dans un véhicule approprié; ces copolymères étant formés de 70 à 90% des monomères hydrophobes et de 5 à 30% des monomères hydrophiles en pourcentages pondéraux et se trouvant dans le vernis à ongles dans une proportion de 10 à 40% du poids de la composition.

2. La composition de la revendication 1 dans laquelle les monomères hydrophobes contiennent un mélange de deux quelconques ou plus des esters des catégories (1), (2), (3) et (4), et de préférence un mélange des esters des catégories (1) et (2).

3. La composition de la revendication 1 ou 2 dans laquelle les monomères hydrophiles comprennent des mélanges d'acrylamides et de méthacrylamides N-oxo-substitués.

4. La composition de la revendication 3 dans laquelle le mélange des monomères hydrophiles contient également des acrylates et méthacrylates d'hydroxyalkyles.

5. La composition de la revendication 3 dans laquelle les monomères hydrophiles comprennent de 50 à 75% en poids, de préférence 75%, des N-oxo-acrylamides et de 25 à 50% en poids, de préférence 25%, des acides carboxyliques α,β-insaturés.

6. La composition de l'une quelconque des revendications précédentes dans laquelle le véhicule est un solvant des copolymères.

7. La composition de la revendication 6 dans laquelle le solvant est choisi parmi les acétates d'éthyle, d'amyle, de butyle, de butyl cellosolve, de cellosolve et de méthyl cellosolve, l'acétone, la méthyléthylcétone, la methylisobutyl-cétone, le butyl cellosolve, le cellosolve, le méthyl cellosolve, les alcools éthylique, isopropylique et butylique, le toluène et les xylènes.

8. La composition de la revendication 6 ou 7 dans laquelle le solvant est un mélange en quantités égales d'acétate de butyle et d'alcool isopropylique.

9. La composition de l'une quelconque des revendications précédentes qui comprend des colorants, des agents de mise en suspension et des charges, et/ou également une ou plusieurs autres résines filmogènes et un plastifiant.

10. La composition de la revendication 9 qui comprend des agents de viscosité choisis parmi le diméthacrylate d'éthylène-glycol, le diméthacrylate de tétraéthylèneglycol, le triméthacrylate de triméthylol propane, le méthacrylate de tétrahydrofurfuryle et le méthacrylate d'allyle, dans une proportion de 0,01 à 0,5% du poids de la composition.

11. La composition de la revendication 3 dans laquelle la proportion des copolymères des monomères hydrophobes et des monomères hydrophiles est de 30 à 40% du poids de la composition.

12. La composition de l'une quelconque des revendications précédentes dans laquelle les monomères hydrophobes constituent une partie principale des copolymères.

13. Une composition de vernis à ongles sans nitrocellulose qui comprend des copolymères de monomères hydrophobes et de monomères hydrophiles, les monomères hydrophobes étant un mélange d'au moins deux esters différents d'acides carboxyliques α,β-insaturés, esters qui sont choisis parmi:

(1) les acrylates et méthacrylates de méthyle, d'éthyle, de propyle et de butyle,

(2) les acrylates et méthacrylates de cyclohexyle, benzyle, isobornyle, adamantyle et furfuryle,

(3) les acrylates et méthacrylates de 2-éthylhexyle, isobutyle, iso-octyle, dodécyle, octadécyle et isodécyle, et

(4) les acrylates et méthacrylates de méthoxyéthyle, éthoxyéthyle, propoxyéthyle, butoxyéthyle et phénoxyéthyle; et

les monomères hydrophiles étant des N-oxo-acrylamides choisis parmi le diacétone-acrylamide le diméthylacrylamide, le butylacrylamide, l'octylacrylamide, l'isobutylméthylacrylamide et le N-(isobutoxyméthyl)-acrylamide;

ces copolymères de monomères hydrophobes et de monomères hydrophiles se trouvant dans un véhicule approprié et comprenant de 70 à 90% des monomères hydrophobes et de 5 à 30% des monomères hydrophiles en pourcentages pondéraux par rapport au poids du copolymère, et la proportion de ces copolymères étant de 10 à 40% du poids de la composition.